# EUROPEAN PATENT APPLICATION

(11) **EP 4 473 928 A1**
(43) Date of publication of application: **11.12.2024**
(21) Application number: 24179746.3
(22) Date of filing: 04.06.2024
(51) Int. Cl.: A61B 18/14, A61B 5/24

(54) **EXPANDABLE BASKET ASSEMBLIES AND EXPANDABLE BASKET ASSEMBLIES WITH ELECTRODE WIRE STRAIN RELIEF**

(30) Priority: 05.06.2023 US 202363506217 P; 01.05.2024 US 202418652292
(71) Applicant: Biosense Webster (Israel) Ltd., 2066717 Yokneam (IL)
(72) Inventor: AGNEW V, William James, Irvine, 92618 (US); VAN NIEKERK, Pieter Emmelius, Irvine, 92618 (US); THALER, Sean D., Irvine, 92618 (US); RODRIGUEZ, Jasson, Irvine, 92618 (US)
(74) Representative: Carpmaels & Ransford LLP

(57) **Abstract**

The disclosed technology includes an expandable spine including a connecting portion that includes a first slit facing an interior space formed by the expandable spine when the expandable spine is in an expanded state, a first electrode attached to the expandable spine and disposed at a distal end of the first slit. a second electrode attached to the expandable spine and disposed proximal to the first electrode and at a proximal end of the first slit, and a wire configured to electrically connect the first and second electrodes and configured to be positioned within the first slit when the expandable spine is in a collapsed state and be positioned outside of the first slit such that the wire travels in a more direct path compared to the path of the connecting portion the first and second electrodes when in the expanded state.

## Description

### CROSS-REFERENCE TO RELATED APPLICATION

The application claims benefit of priority to prior filed U.S. Provisional Patent Application No. 63/506,217, filed June 5, 2023 (Attorney Docket No. 253757.000351), which is hereby incorporated by reference in full herein.

### FIELD

The present invention relates generally to medical devices, and in particular catheters with electrodes, and further relates to, but not exclusively, catheters suitable for use to induce irreversible electroporation (IRE) of cardiac tissues.

### BACKGROUND

Cardiac arrhythmias, such as atrial fibrillation (AF), occur when regions of cardiac tissue abnormally conduct electric signals to adjacent tissue. This disrupts the normal cardiac cycle and causes asynchronous rhythm. Certain procedures exist for treating arrhythmia, including surgically disrupting the origin of the signals causing the arrhythmia and disrupting the conducting pathway for such signals. By selectively ablating cardiac tissue by application of energy via a catheter, it is sometimes possible to cease or modify the propagation of unwanted electrical signals from one portion of the heart to another.

Many current ablation approaches in the art utilize radiofrequency (RF) electrical energy to heat tissue. RF ablation can have certain risks related to thermal heating which can lead to tissue charring, burning, steam pop, phrenic nerve palsy, pulmonary vein stenosis, and esophageal fistula.

Cryoablation is an alternative approach to RF ablation that generally reduces thermal risks associated with RF ablation. Maneuvering cryoablation devices and selectively applying cryoablation, however, is generally more challenging compared to RF ablation; therefore cryoablation is not viable in certain anatomical geometries which may be reached by electrical ablation devices.

Some ablation approaches use irreversible electroporation (IRE) to ablate cardiac tissue using nonthermal ablation methods. IRE delivers short pulses of high voltage to tissues and generates an unrecoverable permeabilization of cell membranes. Delivery of IRE energy to tissues using multi-electrode catheters was previously proposed in the patent literature. Examples of systems and devices configured for IRE ablation are disclosed in U.S. Patent Pub. No. 2021/0169550A1, 2021/0169567A1, 2021/0169568A1, 2021/0161592A1, 2021/0196372A1, 2021/0177503A1, and 2021/0186604A1, each of which are incorporated herein by reference in their entirety.

Regions of cardiac tissue can be mapped by a catheter to identify the abnormal electrical signals. The same or different catheter can be used to perform ablation. Some example catheters include a number of spines with electrodes positioned thereon. The electrodes are generally attached to the spines and secured in place by soldering, welding, or using an adhesive. Due to the small size of the spines and the electrodes, however, soldering, welding, or adhering the electrodes to the spines can be a difficult task, increasing the manufacturing time and cost and the chances that the electrode fails due to an improper bond or misalignment. What is needed, therefore, are systems and methods of attaching an electrode to a spine of a basket assembly without the need for soldering, welding, or using adhesive.

### SUMMARY

There is provided, in accordance with an example of the present invention, an expandable spine assembly may include an expandable spine comprising a connecting portion that may include a first slit facing an interior space formed by the expandable spine when the expandable spine is in an expanded state. A first electrode attached to the expandable spine and disposed at a distal end of the first slit. A second electrode attached to the expandable spine and disposed proximal to the first electrode and at a proximal end of the first slit. A wire configured to electrically connect the first and second electrodes and configured to be positioned within the first slit when the expandable spine is in a collapsed state and be positioned outside of the first slit such that the wire travels in a more direct path compared to the path of the connecting portion the first and second electrodes when in the expanded state.

There is provided, in accordance with an example of the present invention, an expandable basket assembly that may include a plurality of spines extending along a longitudinal axis and configured to bow radially outward from the longitudinal axis when the expandable basket assembly is transitioned from a collapsed form to an expanded form. The expandable basket assembly may further include a plurality of electrodes pairs, each electrode pair of the plurality of electrodes pairs being attached to a spine of the plurality of spines. The expandable basket assembly may further include a plurality of slits facing an interior space formed by the expandable basket assembly when the expandable basket assembly is in the expanded form. The expandable basket assembly may further include a plurality of wires configured to electrically connect the plurality of electrode pairs and configured to be positioned within the plurality of slits when the expandable basket assembly is in a collapsed state and be positioned outside of the plurality of slits such that the plurality of wires travel in a more direct path compared to the path of the plurality of spines connecting the plurality of electrode pairs.

There is provided, in accordance with an example of the present invention, an expandable spine assembly including an expandable spine comprising a connecting portion. The expandable spine assembly may also include a first electrode attached to the expandable spine at a distal end of the connecting portion. The expandable basket assembly may also include a second electrode attached to the expandable spine and disposed proximal to the first electrode and at a proximal end of the connecting portion. The expandable basket assembly may also include a wire configured to electrically connect centers of the first and second electrodes, be disposed separate from the connecting portion, and disposed within an interior space formed by the expandable spine in an expanded state.

### BRIEF DESCRIPTION OF THE DRAWINGS

FIG. 1 is a schematic pictorial illustration of a medical system including a medical probe whose distal end includes a basket assembly with electrodes, in accordance with an embodiment of the present invention;
FIG. 2A is a schematic pictorial illustration showing a perspective view of a medical probe in an expanded form, with wires connecting electrode adjacent ends of electrodes, in accordance with an embodiment of the present invention;
FIG. 2B is a schematic pictorial illustration showing a side view of a medical probe of FIG. 2A in a collapsed form, in accordance with the disclosed technology;
FIG. 2C is a schematic pictorial illustration showing a top view of a spine of a basket assembly in collapsed form with connecting wires placed within a groove within a spine, in accordance with the disclosed technology.
FIG. 2D is a schematic pictorial illustration showing a perspective view of a medical probe in an expanded form, with wires connecting electrode centers, in accordance with an embodiment of the present invention.
FIG. 2E is a schematic pictorial illustration showing a side view of a spine of a basket assembly in expanded form with connecting wires placed within a groove within a spine along an arc length, in accordance with the disclosed technology.
FIG. 2F is a schematic pictorial illustration showing a side view of a spine of a basket assembly in expanded form with connecting wires extended outside of a groove of a spine with a straight length, in accordance with the disclosed technology.
FIGs. 3A and 3B are schematic pictorial illustrations showing exploded views of a tubular shaft and spines of the basket assembly to illustrate how the spines can be assembled together with the tubular shaft, in accordance with an embodiment of the present invention;
FIG. 4A is a schematic pictorial illustration showing a front perspective view of an electrode before being crimped while FIG. 4B is a schematic pictorial illustration showing a front perspective view of an electrode after being crimped, in accordance with an embodiment of the present invention;
FIG. 5A is a schematic pictorial illustration showing a side perspective view of an electrode before being crimped while FIG. 5B is a schematic pictorial illustration showing a side perspective view of an electrode after being crimped, in accordance with an embodiment of the present invention;
FIG. 6A is a schematic pictorial illustration showing a front perspective view of an electrode and a spine before the electrode is crimped to the spine while FIG. 6B is a schematic pictorial illustration showing a front perspective view of an electrode and a spine after the electrode is crimped to the spine, in accordance with an embodiment of the present invention;
FIGs. 7A-7D illustrate side views of electrodes, in accordance with embodiments of the present invention;

### DETAILED DESCRIPTION

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±10% of the recited value, e.g. "about 90%" may refer to the range of values from 81% to 99%. In addition, as used herein, the terms "patient," "host," "user," and "subject" refer to any human or animal subject and are not intended to limit the systems or methods to human use, although use of the subject invention in a human patient represents a preferred embodiment. As well, the term "proximal" indicates a location closer to the operator or physician whereas "distal" indicates a location further away to the operator or physician.

As discussed herein, vasculature of a "patient," "host," "user," and "subject" can be vasculature of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example.

As discussed herein, "operator" can include a doctor, surgeon, technician, scientist, or any other individual or delivery instrumentation associated with delivery of a multi-electrode catheter for the treatment of drug refractory atrial fibrillation to a subject.

As discussed herein, the term "ablate" or "ablation", as it relates to the devices and corresponding systems of this disclosure, refers to components and structural features configured to reduce or prevent the generation of erratic cardiac signals in the cells by utilizing non-thermal energy, such as irreversible electroporation (IRE), sometimes referred to interchangeably as pulsed electric field (PEF) and pulsed field ablation (PFA). Ablating or ablation as it relates to the devices and corresponding systems of this disclosure is used throughout this disclosure in reference to non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation as understood by a person skilled in the relevant art.

As discussed herein, the terms "bipolar" and "unipolar" when used to refer to ablation schemes describe ablation schemes which differ with respect to electrical current path and electric field distribution. "Bipolar" refers to ablation scheme utilizing a current path between two electrodes that are both positioned at a treatment site; current density and electric flux density is typically approximately equal at each of the two electrodes. "Unipolar" refers to ablation scheme utilizing a current path between two electrodes where one electrode having a high current density and high electric flux density is positioned at a treatment site, and a second electrode having comparatively lower current density and lower electric flux density is positioned remotely from the treatment site.

As discussed herein, the terms "tubular" and "tube" are to be construed broadly and are not limited to a structure that is a right cylinder or strictly circumferential in cross-section or of a uniform cross-section throughout its length. For example, the tubular structures are generally illustrated as a substantially right cylindrical structure. However, the tubular structures may have a tapered or curved outer surface without departing from the scope of the present disclosure.

The present disclosure is related to systems, method or uses and devices for IRE ablation of cardiac tissue to treat cardiac arrhythmias. Ablative energies are typically provided to cardiac tissue by a tip portion of a catheter which can deliver ablative energy alongside the tissue to be ablated. Some example catheters include three-dimensional structures at the tip portion and are configured to administer ablative energy from various electrodes positioned on the three-dimensional structures. Ablative procedures incorporating such example catheters can be visualized using fluoroscopy.

Ablation of cardiac tissue using application of a thermal technique, such as radio frequency (RF) energy and cryoablation, to correct a malfunctioning heart is a well-known procedure. Typically, to successfully ablate using a thermal technique, cardiac electropotentials need to be measured at various locations of the myocardium. In addition, temperature measurements during ablation provide data enabling the efficacy of the ablation. Typically, for an ablation procedure using a thermal technique, the electropotentials and the temperatures are measured before, during, and after the actual ablation. RF approaches can have risks that can lead to tissue charring, burning, steam pop, phrenic nerve palsy, pulmonary vein stenosis, and esophageal fistula. Cryoablation is an alternative approach to RF ablation that can reduce some thermal risks associated with RF ablation. However maneuvering cryoablation devices and selectively applying cryoablation is generally more challenging compared to RF ablation; therefore cryoablation is not viable in certain anatomical geometries which may be reached by electrical ablation devices.

While RF ablation and cryoablation, are based on thermal energy transfer to induce local tissue necrosis, the solution of this disclosure can resolve these and other problems by utilizing irreversible electroporation (IRE), sometimes referred to interchangeably as pulsed electric field (PEF) ablation and pulsed field ablation (PFA). IRE as discussed in this disclosure is a non-thermal cell death technology that can be used for ablation of atrial arrhythmias. To ablate using IRE/PEF, biphasic voltage pulses are applied to disrupt cellular structures of myocardium. The biphasic pulses are non-sinusoidal and can be tuned to target cells based on electrophysiology of the cells. In contrast, to ablate using RF, a sinusoidal voltage waveform is applied to produce heat at the treatment area, indiscriminately heating all cells in the treatment area. IRE therefore has the capability to spare adjacent heat sensitive structures or tissues which would be of benefit in the reduction of possible complications known with ablation or isolation modalities. Additionally, or alternatively, monophasic pulses can be utilized.

Electroporation can be induced by applying a pulsed electric field across biological cells to cause reversable (temporary) or irreversible (permanent) creation of pores in the cell membrane. The cells have a transmembrane electrostatic potential that is increased above a resting potential upon application of the pulsed electric field. While the transmembrane electrostatic potential remains below a threshold potential, the electroporation is reversable, meaning the pores can close when the applied pulse electric field is removed, and the cells can self-repair and survive. If the transmembrane electrostatic potential increases beyond the threshold potential, the electroporation is irreversible, and the cells become permanently permeable. As a result, the cells die due to a loss of homeostasis and typically die by apoptosis. Generally, cells of differing types have differing threshold potential. For instance, heart cells have a threshold potential of approximately 500 V/cm, whereas for bone it is 3000 V/cm. These differences in threshold potential allow IRE to selectively target tissue based on threshold potential.

The solution of this disclosure includes systems and methods for applying electrical signals from catheter electrodes positioned in the vicinity of myocardial tissue to generate a pulsed electric field effective to induce electroporation in the myocardial tissue. The systems and methods can be effective to ablate targeted tissue by inducing irreversible electroporation. In some examples, the systems and methods can be effective to induce reversible electroporation as part of a diagnostic procedure. Reversible electroporation occurs when the electricity applied with the electrodes is below the electric field threshold of the target tissue allowing cells to repair. Reversible electroporation does not kill the cells but allows a physician to see the effect of reversible electroporation on electrical activation signals in the vicinity of the target location. Example systems and methods for reversible electroporation is disclosed in U.S. Patent Publication 2021/0162210, the entirety of which is incorporated herein by reference in their entirety.

The pulsed electric field, and its effectiveness to induce reversible and/or irreversible electroporation, can be affected by physical parameters of the system and biphasic pulse parameters of the electrical signal. Physical parameters can include electrode contact area, electrode spacing, electrode geometry, etc. examples presented herein generally include physical parameters adapted to effectively induce reversible and/or irreversible electroporation. Biphasic pulse parameters of the electrical signal can include voltage amplitude, pulse duration, pulse interphase delay, inter-pulse delay, total application time, delivered energy, etc. In some examples, parameters of the electrical signal can be adjusted to induce both reversible and irreversible electroporation given the same physical parameters. Examples of various systems and methods of ablation including IRE are presented in U.S. Patent Publications 2021/0169550A1, 2021/0169567A1, 2021/0169568A1, 2021/0161592A1, 2021/0196372A1, 2021/0177503A1, and 2021/0186604A1, the entireties of each of which are incorporated herein by reference in their entirety.

To deliver pulsed field ablation (PFA) in an IRE (irreversible electroporation) procedure, electrodes should contact the tissue being ablated with a sufficiently large surface area. As described hereinbelow, the medical probe includes a flexible insertion tube having proximal and distal ends, and a basket assembly at the distal end of the flexible insertion tube. The basket assembly includes at least one spine and a plurality of electrodes, each given electrode having a lumen therethrough fitting a given spine. The electrodes are crimped to the spine and locked in place with a locking stub to prevent the electrodes from sliding proximally or distally along the length of the spine.

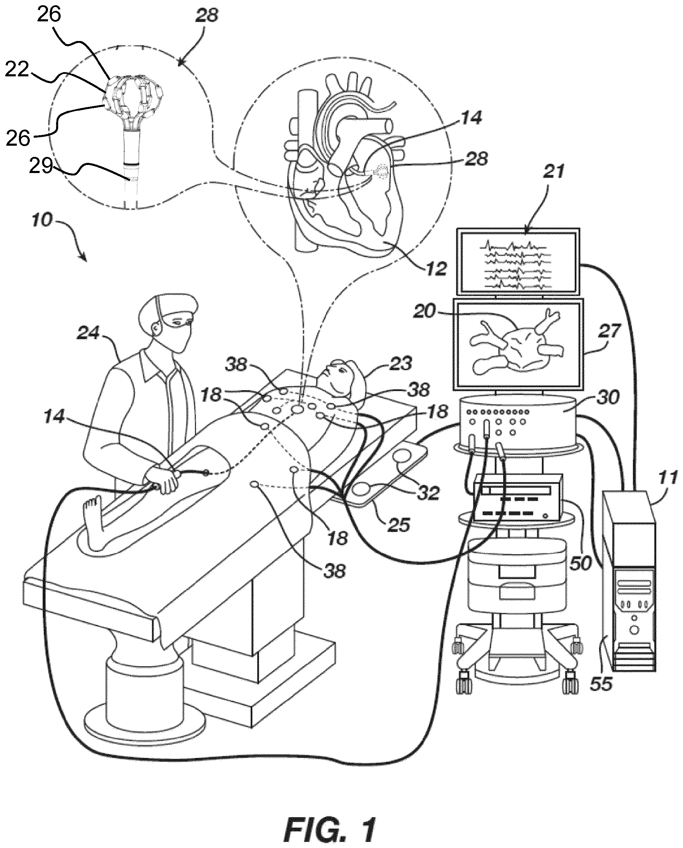

Reference is made to FIG. 1 showing an example catheter-based electrophysiology mapping and ablation system 10. System 10 includes multiple catheters, which are percutaneously inserted by operator 24 through the patient's 23 vascular system into a chamber or vascular structure of a heart 12. Typically, a delivery sheath catheter is inserted into the left or right atrium near a desired location in heart 12. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters dedicated for both sensing and ablating. An example catheter 14 that is configured for sensing IEGM is illustrated herein. Operator 24 brings a basket catheter 28 into contact with the heart wall for sensing a target site in heart 12. For ablation, operator 24 would similarly bring a distal end of an ablation catheter to a target site for ablating.

Catheter 14 is an exemplary catheter that includes one and preferably multiple electrodes 26 optionally distributed over a plurality of spines 22 forming a basket assembly 28 at a distal end and configured to sense the IEGM signals. Catheter 14 may additionally include a position sensor 29 embedded in or near the distal tip for tracking position and orientation of basket assembly 28. Optionally and preferably, position sensor 29 is a magnetic based position sensor including three magnetic coils for sensing three-dimensional (3D) position and orientation.

Magnetic based position sensor 29 may be operated together with a location pad 25 including a plurality of magnetic coils 32 configured to generate magnetic fields in a predefined working volume. Real time position of basket assembly 28 of catheter 14 may be tracked based on magnetic fields generated with location pad 25 and sensed by magnetic based position sensor 29. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091, each of which are incorporated herein by reference in their entirety.

System 10 includes one or more electrode patches 38 positioned for skin contact on patient 23 to establish location reference for location pad 25 as well as impedance-based tracking of electrodes 26. For impedance-based tracking, electrical current is directed toward electrodes 26 and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182, each of which are incorporated herein by reference in their entirety.

A recorder 11 displays electrograms 21 captured with body surface ECG electrodes 18 and intracardiac electrograms (IEGM) captured with electrodes 26 of catheter 14. Recorder 11 may include pacing capability for pacing the heart rhythm and/or may be electrically connected to a standalone pacer.

System 10 may include an ablation energy generator 50 that is adapted to conduct ablative energy to one or more of electrodes at a distal tip of a catheter configured for ablating. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof. The signals may be biphasic or monophasic.

Patient interface unit (PIU) 31 is an interface configured to establish electrical communication between catheters, electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 31 additionally includes processing capability for implementing real-time computations of location of the catheters and for performing ECG calculations.

Workstation 55 includes memory, processor unit with memory or storage with appropriate operating software loaded therein, and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of multiple catheters within the heart chamber, and (5) displaying on display device 27 sites of interest such as places where ablation energy has been applied. One commercial product embodying elements of the system 10 is available as the CARTO^{™} 3 System, available from Biosense Webster, Inc., 31 Technology Drive, Suite 200, Irvine, CA 92618, USA.

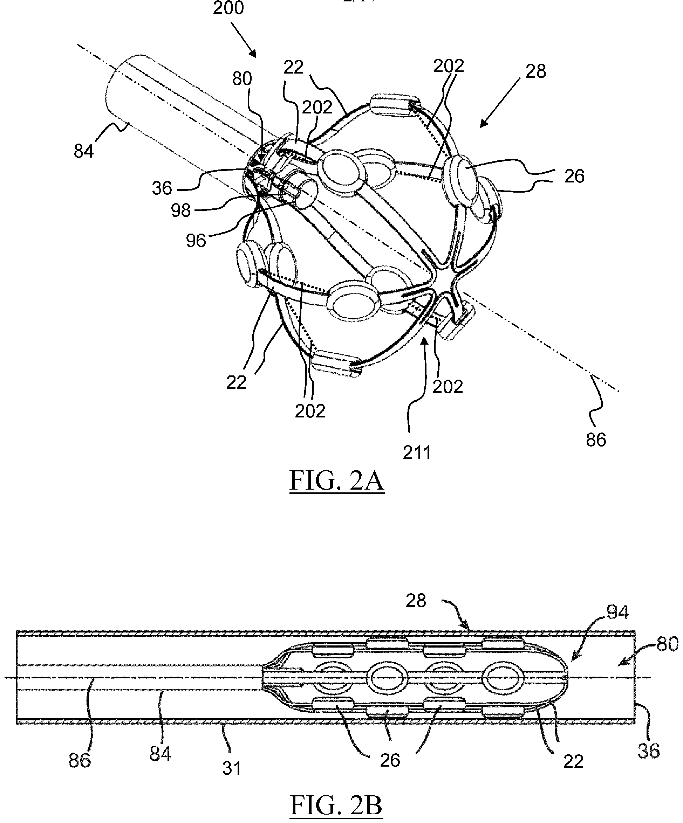

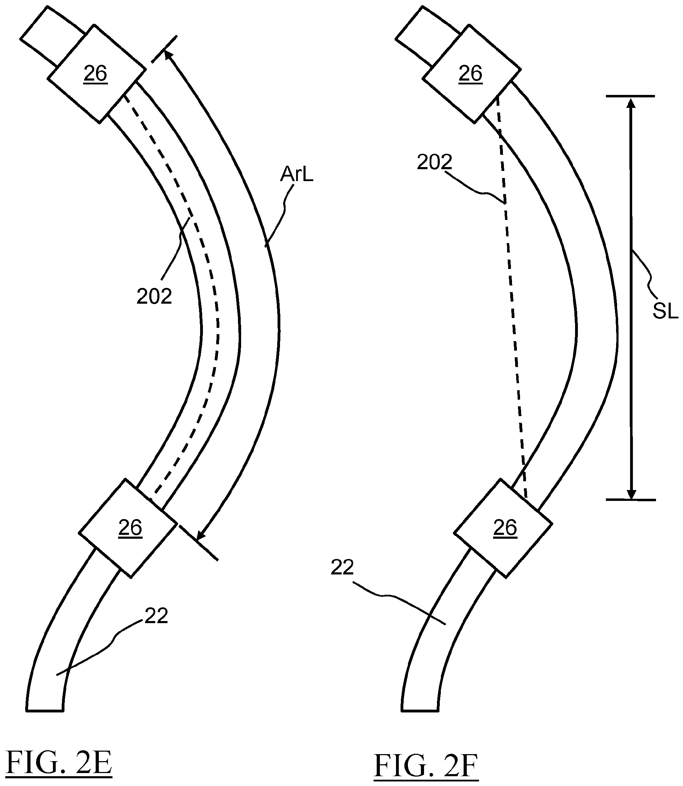

Fig. 2A is a schematic pictorial illustration showing a perspective view of a medical probe 200 having a basket assembly 28 in an expanded form when unconstrained, such as by being advanced out of an insertion tube lumen 80 at a distal end 36 of an insertion tube 31. Fig. 2B shows the basket assembly in a collapsed form within insertion tube 31. In the expanded form (Fig. 2A), the spines 22 bow radially outwardly from a longitudinal axis 86 of the medical probe 200 and in the collapsed form (Fig. 2B) the spines are arranged generally along the longitudinal axis 86 of the medical probe 200.

As shown in Fig. 2A, basket assembly 28 includes a plurality of flexible spines 22 that are formed at the end of a tubular shaft 84 and are connected at both ends. During a medical procedure, medical professional 24 can deploy basket assembly 28 by extending tubular shaft 84 from insertion tube 31 causing the basket assembly 38 to exit the insertion tube and transition to the expanded form. Spines 22 may have elliptical (e.g., circular) or rectangular (that may appear to be flat) cross-sections, and include a flexible, resilient material (e.g., a shape-memory alloy such as nickel-titanium (also known as Nitinol), cobalt chromium, stainless steel, titanium, or any other suitable material or combinations of materials).

In embodiments described herein, electrodes 26 can be configured to deliver ablation energy (RF and/or IRE) to tissue in heart 12. For example, the electrodes 26 can be configured to deliver 20-30 amps of electrical current to cardiac tissue to facilitate ablation of the cardiac tissue. Furthermore, the electrodes 26 can be configured for bipolar or unipolar ablation schemes depending on the particular application. Alternatively, or in addition, the electrodes can be used to determine the location of basket assembly 28 and/or to measure a physiological property such as local surface electrical potentials at respective locations on tissue in heart 12.

Examples of materials ideally suited for forming electrodes 26 include gold, platinum, and palladium (and their respective alloys). These materials also have high thermal conductivity which allows the minimal heat generated on the tissue (i.e., by the ablation energy delivered to the tissue) to be conducted through the electrodes to the back side of the electrodes (i.e., the portions of the electrodes on the inner sides of the spines), and then to the blood pool in heart 12.

Basket assembly 28 has a distal end 94 and includes a stem 96 that extends longitudinally from a distal end 36 of shaft 84 towards distal end 94 of basket assembly 28. C control console or work station 55 may include an irrigation module (not illustrated) that controls the delivery of irrigation fluid to distal end 36. Stem 96 includes multiple spray ports 98, wherein each given spray port 98 can be angled to aim delivery of the irrigation fluid to either a given electrode 26 or to tissue in heart 12.

Since electrodes 26 do not include spray ports that deliver irrigation fluid, the configuration described hereinabove enables heat to be transferred from the tissue (i.e., during an ablation procedure) to the portion of the electrodes 26 on the inner side of the spines 22, and the electrodes 26 can be cooled by aiming the irrigation fluid, via spray ports 98, at the portion of the electrodes 26 on the inner side of the spines 22.

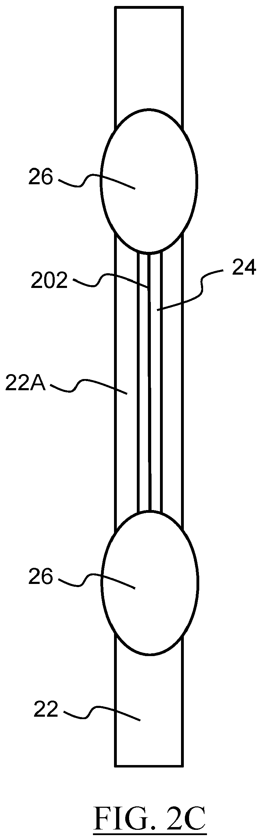

As shown in FIGs. 2A-2F, the spines 22 of the basket assembly 28 include slits 24 (see FIG. 2C) configured to house wires 202 in a collapsed state (see FIGs. 2B and 2C) connecting adjacent ends of electrodes 26. The wires 202 are configured to electrically connect the first and second electrodes 26 and are connected to a spine 22 and configured to be positioned within the first slit 24 when the expandable spine 22 is in a collapsed state and be positioned outside of the first slit such that the wire travels in a more direct path compared to the path of the connecting portion 22A (see FIG. 2C) the first and second electrodes 26 when in the expanded state. An arc length ArL of the connecting portion 22A is greater than a length SL of the wire 202 between the first and second electrodes 26 when the expandable spine 22 is in the expanded state. In some embodiments, the basket assembly 28 may include an electrically insulative jacket disposed between the first and second electrodes 26 and the expandable spine, thereby electrically isolating the first and second electrodes from the expandable spine. In this case, the electrically insulative jacket includes a second slit that corresponds with the first slit and comprises polyether keytone (PEEK), liquid crystal polymer (LCP), or both.

Put another way, an expandable basket 28 assembly may include a plurality of spines 22 extending along a longitudinal axis 86 and configured to bow radially outward from the longitudinal axis 86 when the expandable basket assembly 28 is transitioned from a collapsed form to an expanded form. The expandable basket assembly may include a plurality of electrodes pairs 26, each electrode 26 pair of the plurality of electrodes 26 pairs being attached to a spine 22 of the plurality of spines 22. A plurality of slits 24 facing an interior space formed by the expandable basket assembly 28 when the expandable basket assembly 28 is in the expanded form. A plurality of wires 202 configured to electrically connect the plurality of electrode 26 pairs and configured to be positioned within the plurality of slits 24 when the expandable basket assembly 28 is in a collapsed state and be positioned outside of the plurality of slits 24 such that the plurality of wires 202 travel in a more direct path compared to the path of the plurality of spines 22 connecting the plurality of electrode 26 pairs.

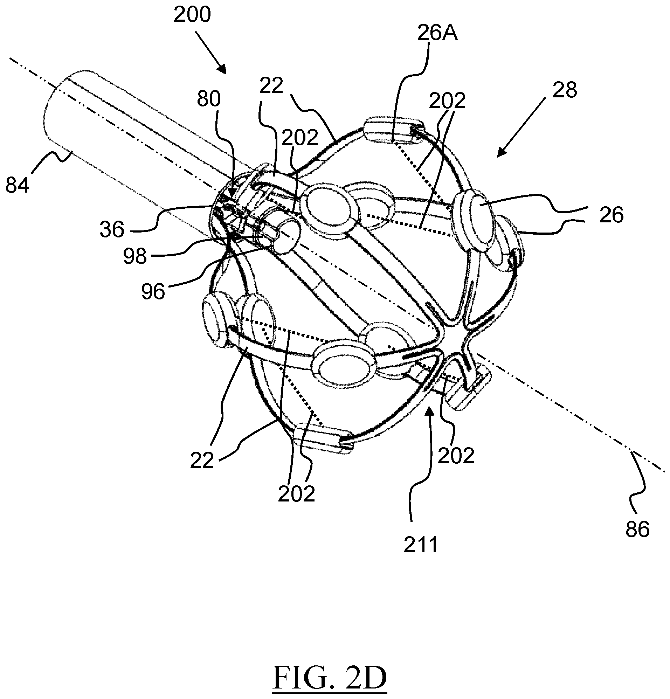

FIG.2D is the same as FIG. 2A except the wires 202 of FIG. 2D connect center 26A of adjacent electrodes 26 disposed on a spine 22.

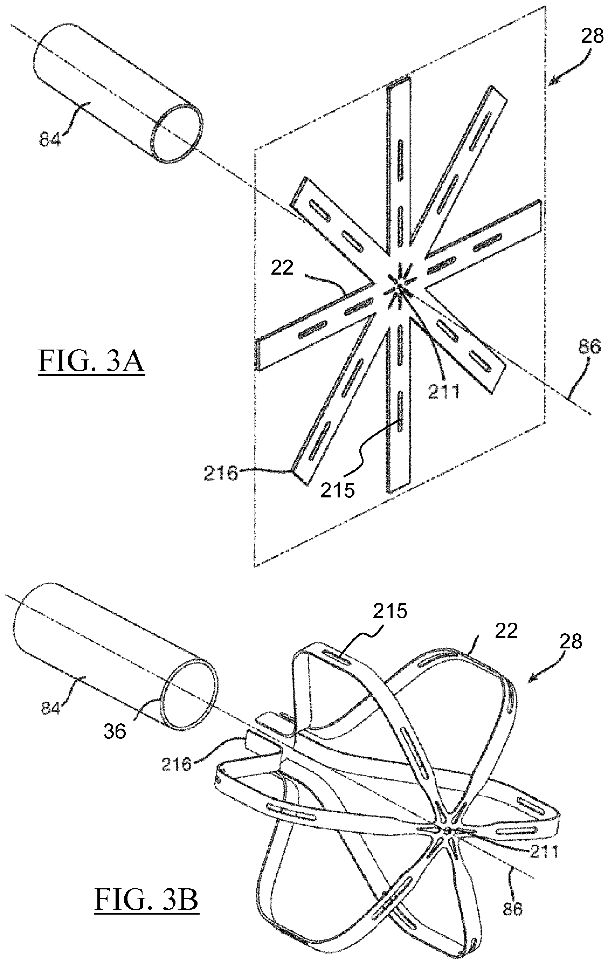

Figs. 3A and 3B are schematic pictorial illustrations showing exploded views of a tubular shaft 84 and spines 22 of the basket assembly 28 to provide one example of how the spines 22 can be assembled together with the tubular shaft 84, in accordance with an embodiment of the present invention. As shown in Fig. 3A, the spines 22 can form a spine assembly 28. The spines 22 can be formed from a single sheet of planar material to form a generally star shape. In other words, the spines 22 can be formed from the single sheet of planar material such that the spines 22 converge toward a central intersection 211. The intersection 211 can be a solid piece of material or include one or more apertures.

The spines 22 can be folded or otherwise bent such that a proximal end 216 of the spines 22 can be inserted into the distal end 36 (see Figs. 2A and 2D) of the tubular shaft 84 as shown in Fig. 3B. Although not shown in Figs. 3A and 3B, it will be appreciated that the electrodes 26 can be attached to the spines 22 before the spines are inserted into the tubular shaft 84 to form the basket assembly 28. As stated previously, the spines 22 can include a flexible, resilient material (e.g., a shape-memory alloy such as nickel-titanium, also known as Nitinol) that can enable the basket assembly 28 to transition from its collapsed form (as shown in Fig. 2B) to its expanded form (as shown in Fig. 2A) when the basket assembly 28 is deployed from the insertion tube 31.

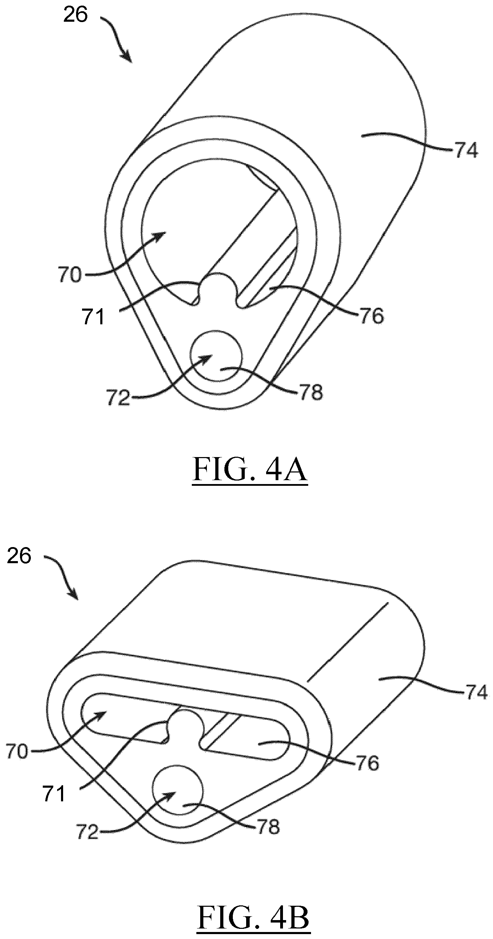

The spines 22 can each define an aperture 215 that can be sized and positioned to receive a locking stub 71 of the electrode 26 as will be described in greater detail herein. By receiving the locking stub 71, the aperture 215 can help to prevent the electrode 26 from sliding proximally or distally along the length of the spine 22. In other words, the aperture 215 can help to secure the electrode 26 to the spine 22 when the locking stub 71extends through the aperture 215, without requiring a weld, a fastener, adhesive, or other fastening devices or methods. As a non-limiting example, the spines 22 can include at least two apertures 215 positioned on a length of the spine 22 between a proximal end of the spine 22 and a distal end of the spine 22. In this way, the basket assembly 28 can have at least two electrodes 26 along each length of spine 22 extending between the proximal end to the distal end of the basket assembly 28 such that the basket assembly 28 can have a total of twelve electrodes. The aperture 215 can be arranged to receive an electrode (e.g., Figs. 4A, 4B, 5A, 5B, 6A, 6B, 7A-7D) and retain such electrode via a mechanical lock between the electrode and the spine by virtue of aperture 215.

As will be appreciated by one skilled in the art with the benefit of this disclosure, the basket assembly 28 shown in Figs. 2A-3B having spines 22 formed from a single sheet of planar material and converging at a central intersection is offered merely for illustrative purposes and the disclosed technology can be applicable to other configurations of basket assemblies 28. For example, the disclosed technology can be applicable to basket assemblies 28 formed from a single spine 22 or multiple spines 22 with each spine 22 being attached at both ends. In other examples, the basket assembly 28 can include a central hub connecting the multiple spines 22 together at a distal end 94 of the basket assembly 28. In yet other examples, the basket assembly 28 can include a single spine 22 configured to form a spiral, multiple spines 22 configured to form a spiral, multiple spines 22 configured to form a tripod or multiple tripods, or any other shape of basket assembly 28. As well, the spine assembly 28 can be formed by laser cutting a cylindrical hollow stock material whereby the laser is mounted for rotation about the longitudinal axis (and translation thereto) of the cylindrical stock while cutting. Thus, although Figs. 2A-3B illustrate a specific configuration of basket assembly 28, the disclosed technology should not be construed as so limited.

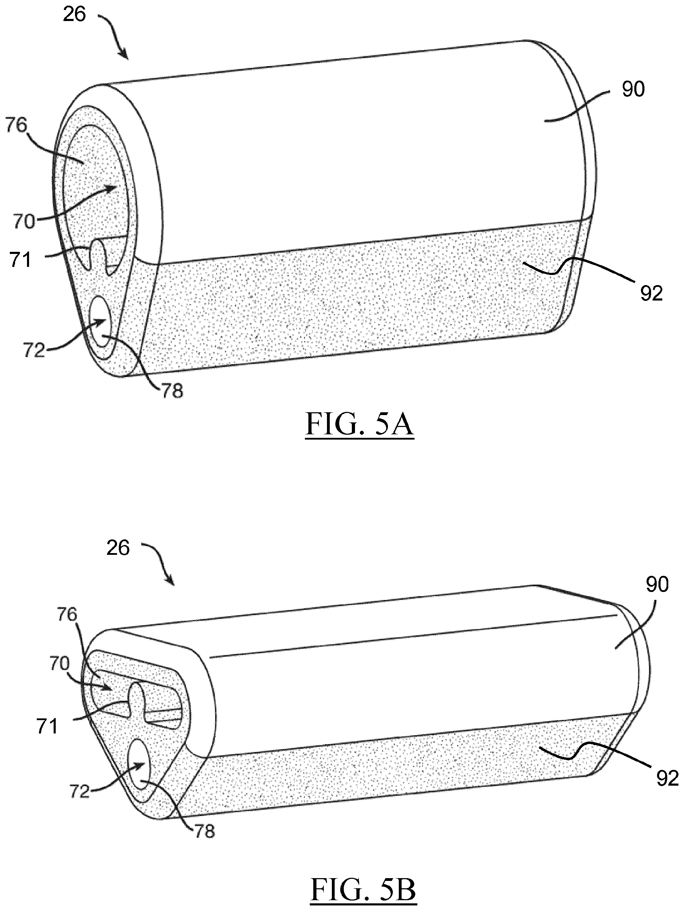

Fig. 4A is a schematic pictorial illustration showing a front perspective view of an electrode 26 before being crimped while Fig. 4B is a schematic pictorial illustration showing a front perspective view of an electrode 26 after being crimped, in accordance with an embodiment of the present invention. Similarly, Fig. 5A is a schematic pictorial illustration showing a side perspective view of an electrode 26 before being crimped while Fig. 5B is a schematic pictorial illustration showing a side perspective view of an electrode 26 after being crimped, in accordance with an embodiment of the present invention. As shown in Figs. 4A-5B, the electrode 26 can have an elongated body that has a cross sectional shape that extends along the length of the electrode 26. The electrode 26 can be made from a malleable and conductive material such as gold, platinum, and palladium (and their respective alloys) as described previously. As will be appreciated, by forming the electrode 26 from a malleable material, the electrode 26 can be crimped or otherwise plastically deformed to transition from a first shape (i.e., Figs. 4A and 5A) that is uncrimped to a second shape (i.e., Figs. 4B and 5B) that is crimped. As will be described in greater detail herein, the electrode 26 can be crimped around a spine 22 to secure the electrode 26 to the spine 22 without requiring solder, weld, or adhesives.

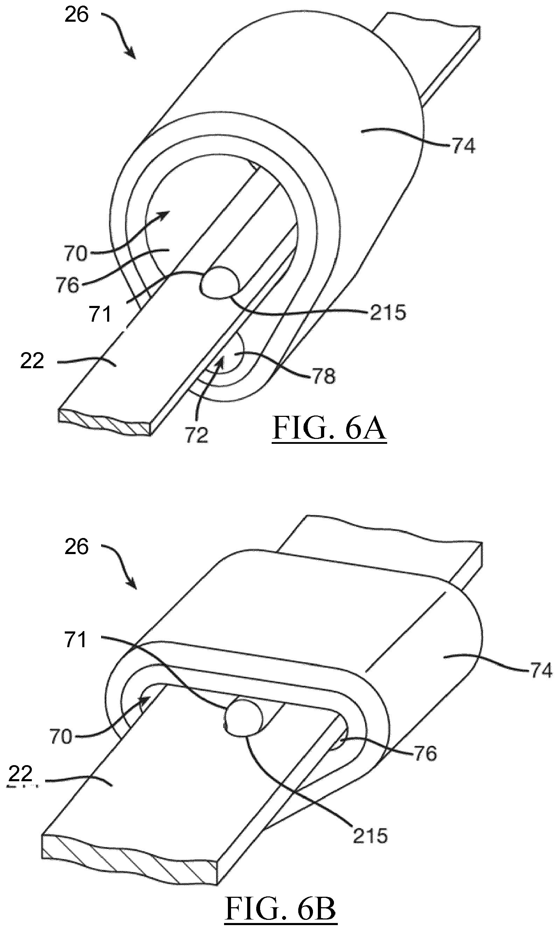

The electrode 26 can define a first lumen 70 and a second lumen 72 which both extend through an elongated body of the electrode 26 from a first end to a second end of the electrode 26. The electrode 26 can have an outer surface 74 facing outwardly from the electrode 26, a first inner surface 76 facing inwardly toward the electrode 26 where the first lumen 70 is formed through the electrode 26, and a second inner surface 78 facing inwardly toward the electrode 26 where the second lumen 72 is formed through the electrode. The first lumen 70 can be sized and configured to receive a spine 22, as shown in Fig. 6A, such that the spine 22 can pass through the first lumen 70. Although not shown, the second lumen 72 can be sized to receive a wire of the medical probe 22 such that the electrode 26 can be attached to the wire. The wire can be electrically insulated from the spine 22. In some examples, the first lumen 70 and/or the second lumen 72 can each pass through the electrode 26 in a generally longitudinal direction of the electrode 26. In other examples, the first lumen 70 and/or the second lumen 72 can pass through the electrode 26 in a generally transverse direction of the electrode 26. Furthermore, the first lumen 70 and/or the second lumen 72 can be positioned in the electrode 26 nearer a bottom surface, nearer at top surface, or nearer a middle of the electrode 26 depending on the particular configuration.

By including a first lumen 70 that is sized to receive the spine 22 of the medical probe 22, the disclosed technology can ensure the electrodes 26 are secured to the spines 22 and prevented from breaking free. Thus, even if the electrodes 26 become dislodged, the electrodes 26 will remain attached to the spines 22 by nature of the strut 430 passing through the first lumen 70.

The electrode 26 can further include a locking stub 80 that can extend inwardly into the first lumen 70. The locking stub 80 can extend longitudinally along the length of the electrode 26 from a first end of the electrode 26 to a second end of the electrode 26. The locking stub 80 can be sized to extend at least partially through an aperture 215 of the spines 22 when the electrode 26 is coupled to the spine 22.

As illustrated in Figs. 4A-6B, the electrode 26 can be crimped from a first shape (i.e., as illustrated in Figs. 4A, 5A, and 6A) to a second shape (i.e., as illustrated in Figs. 4B, 5B, and 6B). For example, the electrodes 26 can be crimped toward a longitudinal axis of the elongated body of the electrode 26 and have a substantially rounded outer surface 74 prior to being crimped and a substantially flat outer surface 74 after being crimped. The electrodes 26 can be crimped by using a tool to press the electrode 26 until the electrode 26 plastically deforms from the first shape to the second shape. As illustrated in Figs. 6A and 6B, a spine 22 can be inserted into the electrode 26 and an aperture 215 of the spine 22 can be aligned with the locking stub 80 of the electrode 26. Once the locking stub 80 is aligned with the aperture 215, the electrode 26 can be crimped until a distance between the locking stub 80 and the first lumen inner surface 76 is reduced and the spine 22 is secured in place. In other words, the spine 22 can be inserted into the first lumen 70 and then the electrode 26 can be crimped until electrode 26 is tightly secured around the spine 22. When the electrode 26 is crimped around the spine 22, the locking stub 80 can extend through the aperture 215 of the spine 22 and the electrode 26 can be prevented from moving proximally or distally along the length of the spine 22. Stated otherwise, once the electrode 26 is crimped to spine 26, the locking stub 80 can contact an inner surface of the aperture 215 of the spine 22 and the first lumen inner surface 76 can contact the spine 22 such that the spine 22 cannot be removed from the electrode 26 without deforming the electrode 26. In some examples, the locking stub 80 can form an interference fit with the spine 22 at the aperture 215 to secure the electrode 26 in place.

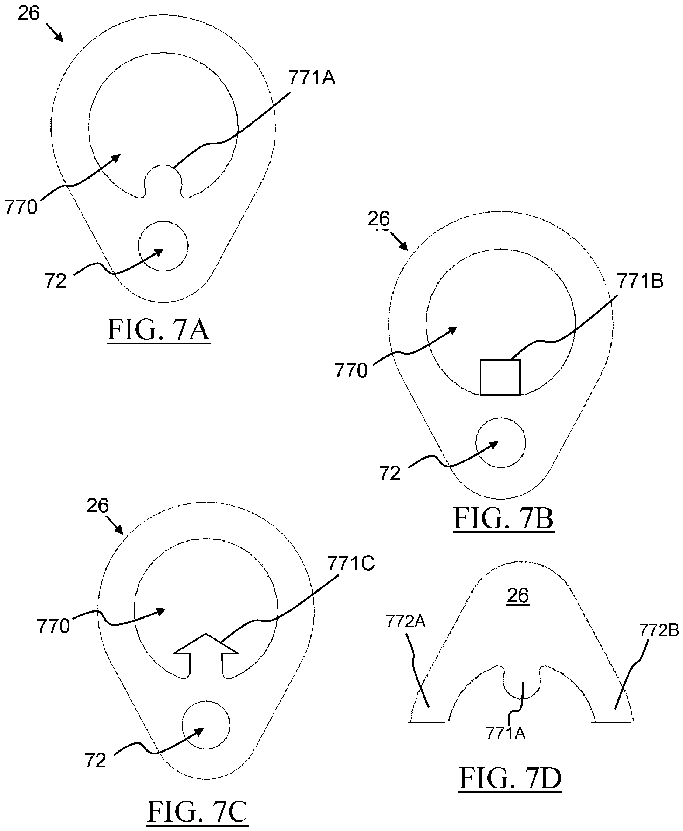

Figs. 7A-7D illustrate side views of electrodes 26 having locking stubs 780A-780C of different shapes. As shown in Fig. 7A, a locking stub 771A can comprise a generally rounded or circular cross section. Alternatively, as shown in Fig. 7B, a locking stub 771B can comprise a generally rectangular cross section or, as shown in Fig. 7C, a locking stub 771C can comprise at least a portion that has a generally triangular shape. As will be appreciated, a generally rounded locking stub 771A can help to permit the locking stub 771A to slide into the aperture 215 of the spine 22 and form an interference fit with the spine 215. Alternatively, a locking stub 771B having a generally rectangular shape may be able to form a tighter interference fit. Furthermore, a locking stub 771 C having a generally triangular shape may be able to lock the spine 22 onto the locking stub 71 with the triangular shape to further secure the electrode 26 to the spine 22. In Fig. 7D, the active electrode surface 26 is now a bulging member with two side prongs 772A and 772B so that the retainer 771A can be inserted into aperture 215 and the electrode 26A is swaged or crimped over the spine member 22. In an alternative arrangement, side prongs 772A and 772B can be eliminated so that only retainer 771A project into the aperture 215 and the electrode 26A can be crimped into a substantially three-dimensional trapezoid (i.e., a rounded trapezoidal prism or rounded and truncated pyramid). As will be appreciated, Figs. 7A-7D are offered for explanatory purposes and the disclosed technology should not limited to the specific examples of locking stubs 771A-771C illustrated and described herein. For example, the locking stub 71 can include a generally trapezoidal, pentagonal, hexagonal, octagonal, elliptical, cross, curved, or other suitable shape. Furthermore, the locking stub 71 can have a changing cross section along the length of the locking stub 71 from one end of the locking stub 71 to another end of the locking stub 71. For example, the locking stub 71 can have a thicker cross section at either end of the locking stub 71 or a thicker cross section at the middle of the locking stub 71. Furthermore, although described as extending from one side of the electrode 26 to a second side of the electrode 26, the locking stub 71 can extend less than the full length of the electrode 26 or may have a portion removed between the first end and second end (e.g., the locking stub 71 can have portions of material at either end of the electrode 26 with no material at the middle of the electrode 26). Further still, although a single locking stub 71 is shown and described herein, the electrode 26 can include more than one locking stub 71 and the locking stub 71 can extend into the first lumen 70 from a portion of the first lumen 70 other than the lower portion of the lumen 70 (e.g., an upper portion of the lumen 70). Accordingly, the disclosed technology should not be construed as limited to the particular configurations shown and described herein.

Returning now to Figs. 5A and 5B, the electrode 26 can include a conductive portion 90 and an insulative portion 92. The conductive portion 90 can be configured to permit electricity to pass therethrough (i.e., for ablation or mapping of the cardiac tissue) and can face generally outward from the basket assembly 71 to face cardiac tissue when in use. The insulative portion 92, on the other hand, can be configured to prevent electricity from passing therethrough to help electrically isolate the spine 22 from the electrode 26. In this way, the insulative portion 92 can prevent a short circuit from forming between the electrode 26 and the spine 22. Alternatively, or in addition, the spine 22 can be made from an insulative material or have an insulative coating or sleeve added thereto to electrically isolate the electrode 26 from the spine 22. As yet another example, an electrically insulating material can be placed between the electrode 26 and the spine 22 to electrically isolate the electrode 26 from the spine 22.

As will be appreciated by one skilled in the art, The embodiments described above are cited by way of example, and the present invention is not limited by what has been particularly shown and described hereinabove. Rather, the scope of the invention includes both combinations and sub combinations of the various features described hereinabove, as well as variations and modifications thereof which would occur to persons skilled in the art upon reading the foregoing description and which are not disclosed in the prior art.

In some examples, disclosed devices (e.g., spines, basket assemblies, electrodes, and/or medical probes) may involve one or more of the following clauses:
Clause 1: An expandable spine assembly comprising: an expandable spine comprising a connecting portion that comprises a first slit facing an interior space formed by the expandable spine when the expandable spine is in an expanded state; a first electrode attached to the expandable spine and disposed at a distal end of the first slit; a second electrode attached to the expandable spine and disposed proximal to the first electrode and at a proximal end of the first slit; and a wire configured to electrically connect the first and second electrodes and configured to be positioned within the first slit when the expandable spine is in a collapsed state and be positioned outside of the first slit such that the wire travels in a more direct path compared to the path of the connecting portion the first and second electrodes when in the expanded state.
Clause 2: The expandable spine assembly according to Clause 1, wherein an arc length of the connecting portion is greater than a length of the wire between the first and second electrodes when the expandable spine is in the expanded state.
Clause 3: The expandable spine according to Clause 1, comprising nitinol, cobalt chromium, stainless steel, titanium, or combinations thereof.
Clause 4: The expandable spine assembly according to Clause 1, wherein in the expandable spine is configured to curve away from the interior space when in the expanded state.
Clause 5: The expandable spine assembly according to Clause 1, further comprising an electrically insulative jacket disposed between the first and second electrodes and the expandable spine, thereby electrically isolating the first and second electrodes from the expandable spine.
Clause 6: The expandable spine assembly according to Clause 5, wherein the electrically insulative jacket comprises a second slit that corresponds with the first slit and comprises polyether keytone (PEEK), liquid crystal polymer (LCP), or both.
Clause 7: The expandable spine assembly according to Clause 1, wherein the first and second electrodes are coupled to the expandable spine and the first and second electrodes each define a lumen so that the expandable spine extends through the respective lumens.
Clause 8: The expandable spine assembly according to Clause 1, wherein the first and second electrodes are configured to deliver electrical pulses for irreversible electroporation, the electrical pulses having a peak voltage of at least 900 volts (V).
Clause 9: An expandable basket assembly comprising: a plurality of spines extending along a longitudinal axis and configured to bow radially outward from the longitudinal axis when the expandable basket assembly is transitioned from a collapsed form to an expanded form; a plurality of electrodes pairs, each electrode pair of the plurality of electrodes pairs being attached to a spine of the plurality of spines; a plurality of slits facing an interior space formed by the expandable basket assembly when the expandable basket assembly is in the expanded form; and a plurality of wires configured to electrically connect the plurality of electrode pairs and configured to be positioned within the plurality of slits when the expandable basket assembly is in a collapsed state and be positioned outside of the plurality of slits such that the plurality of wires travel in a more direct path compared to the path of the plurality of spines connecting the plurality of electrode pairs.
Clause 10: The expandable basket assembly according to Clause 9, wherein at least one of the plurality of spines comprises a connecting portion disposed between a pair of electrodes of the plurality of electrode pairs, the connecting portion comprising a slit configured to receive a wire of the plurality of wires when the expandable basket assembly is in a collapsed state.
Clause 11: The expandable basket assembly according to Clause 10, wherein an arc length of the connecting portion is greater than a length of the wire when the expandable basket assembly is in the expanded form.
Clause 12: The expandable basket assembly according to Clause 9, wherein at least one spine of the plurality of spines comprises nitinol, cobalt chromium, stainless steel, titanium, or combinations thereof.
Clause 13: The expandable basket assembly according to Clause 9, wherein in the plurality of spines comprise form an approximately spherical shape or an approximately oblate-spheroid shape.
Clause 14: The expandable basket assembly according to Clause 9, wherein the plurality of electrode pairs are coupled to respective spines of the plurality of spines and each define a lumen so that the a respective spine extends through the lumen.
Clause 15: The expandable basket assembly according to Clause 9, wherein the plurality of electrode pairs are configured to deliver electrical pulses for irreversible electroporation, the electrical pulses having a peak voltage of at least 900 volts (V).
Clause 16: An expandable spine assembly comprising: an expandable spine comprising a connecting portion; a first electrode attached to the expandable spine at a distal end of the connecting portion; a second electrode attached to the expandable spine and disposed proximal to the first electrode and at a proximal end of the connecting portion; and a wire configured to electrically connect centers of the first and second electrodes, be disposed separate from the connecting portion, and disposed within an interior space formed by the expandable spine in an expanded state.
Clause 17: The expandable spine assembly according to Clause 16, wherein an arc length of the connecting portion is greater than a length of the wire between the first and second electrodes when the expandable spine is in the expanded state.
Clause 18: The expandable spine assembly according to Clause 16, comprising nitinol, cobalt chromium, stainless steel, titanium, or combinations thereof.
Clause 19: The expandable spine assembly according to Clause 16, wherein in the expandable spine is configured to curve away from the interior space when in the expanded state.
Clause 20: The expandable spine assembly according to Clause 16, wherein the first and second electrodes are coupled to the expandable spine and the first and second electrodes each define a lumen so that the expandable spine extends through the lumen.

## Claims

1. An expandable spine assembly comprising:
an expandable spine comprising a connecting portion that comprises a first slit facing an interior space formed by the expandable spine when the expandable spine is in an expanded state;
a first electrode attached to the expandable spine and disposed at a distal end of the first slit;
a second electrode attached to the expandable spine and disposed proximal to the first electrode and at a proximal end of the first slit; and
a wire configured to electrically connect the first and second electrodes and configured to be positioned within the first slit when the expandable spine is in a collapsed state and be positioned outside of the first slit such that the wire travels in a more direct path compared to the path of the connecting portion the first and second electrodes when in the expanded state.

2. The expandable spine assembly according to Claim 1, wherein an arc length of the connecting portion is greater than a length of the wire between the first and second electrodes when the expandable spine is in the expanded state.

3. The expandable spine assembly according to Claim 1, wherein in the expandable spine is configured to curve away from the interior space when in the expanded state.

4. The expandable spine assembly according to Claim 1, further comprising an electrically insulative jacket disposed between the first and second electrodes and the expandable spine, thereby electrically isolating the first and second electrodes from the expandable spine, optionally wherein the electrically insulative jacket comprises a second slit that corresponds with the first slit and comprises polyether keytone (PEEK), liquid crystal polymer (LCP), or both.

5. The expandable spine assembly according to Claim 1, wherein the first and second electrodes are (i) coupled to the expandable spine and the first and second electrodes each define a lumen so that the expandable spine extends through the respective lumens, or (ii) configured to deliver electrical pulses for irreversible electroporation, the electrical pulses having a peak voltage of at least 900 volts (V).

6. An expandable basket assembly comprising:
a plurality of spines extending along a longitudinal axis and configured to bow radially outward from the longitudinal axis when the expandable basket assembly is transitioned from a collapsed form to an expanded form;
a plurality of electrodes pairs, each electrode pair of the plurality of electrodes pairs being attached to a spine of the plurality of spines;
a plurality of slits facing an interior space formed by the expandable basket assembly when the expandable basket assembly is in the expanded form; and
a plurality of wires configured to electrically connect the plurality of electrode pairs and configured to be positioned within the plurality of slits when the expandable basket assembly is in a collapsed state and be positioned outside of the plurality of slits such that the plurality of wires travel in a more direct path compared to the path of the plurality of spines connecting the plurality of electrode pairs.

7. The expandable basket assembly according to Claim 6, wherein at least one of the plurality of spines comprises a connecting portion disposed between a pair of electrodes of the plurality of electrode pairs, the connecting portion comprising a slit configured to receive a wire of the plurality of wires when the expandable basket assembly is in a collapsed state, optionally wherein an arc length of the connecting portion is greater than a length of the wire when the expandable basket assembly is in the expanded form.

8. The expandable basket assembly according to Claim 6, wherein at least one spine of the plurality of spines comprises nitinol, cobalt chromium, stainless steel, titanium, or combinations thereof.

9. The expandable basket assembly according to Claim 6, wherein in the plurality of spines comprise form an approximately spherical shape or an approximately oblate-spheroid shape.

10. The expandable basket assembly according to Claim 6, wherein the plurality of electrode pairs (i) are coupled to respective spines of the plurality of spines and each define a lumen so that the a respective spine extends through the lumen, or (ii) are configured to deliver electrical pulses for irreversible electroporation, the electrical pulses having a peak voltage of at least 900 volts (V).

11. An expandable spine assembly comprising:
an expandable spine comprising a connecting portion;
a first electrode attached to the expandable spine at a distal end of the connecting portion;
a second electrode attached to the expandable spine and disposed proximal to the first electrode and at a proximal end of the connecting portion; and
a wire configured to electrically connect centers of the first and second electrodes, be disposed separate from the connecting portion, and disposed within an interior space formed by the expandable spine in an expanded state.

12. The expandable spine assembly according to Claim 11, wherein an arc length of the connecting portion is greater than a length of the wire between the first and second electrodes when the expandable spine is in the expanded state.

13. The expandable spine according to Claim 1 or the expandable spine assembly according to Claim 11, comprising nitinol, cobalt chromium, stainless steel, titanium, or combinations thereof.

14. The expandable spine assembly according to Claim 11, wherein in the expandable spine is configured to curve away from the interior space when in the expanded state.

15. The expandable spine assembly according to Claim 11, wherein the first and second electrodes are coupled to the expandable spine and the first and second electrodes each define a lumen so that the expandable spine extends through the lumen.
